# FASCICULE DE BREVET EUROPEEN

(11) **EP 0 586 649 B1**
(45) Date de publication et mention de la délivrance du brevet: **14.01.1998**
(21) Numéro de dépôt: 93905410.2
(22) Date de dépôt: 19.02.1993
(51) Int. Cl.: G01N 33/543

(54) **Capteur moléculaire**
Molekularsensor
Molecular sensor

(30) Priorité: 28.02.1992 FR 9202367
(43) Date de publication de la demande: 16.03.1994
(73) Titulaire: THOMSON-CSF, 75008 Paris (FR)
(72) Inventeur: DUBOIS, Jean-Claude, F-92400 Courbevoie (FR); GREMILLET, Jacques, F-92400 Courbevoie (FR); LE GOFFIC, François, F-92400 Courbevoie (FR)
(74) Mandataire: Guérin, Michel
(86) Numéro de dépôt international: FR9300169
(87) Numéro de publication internationale: WO9317339

(56) Documents cités:
- WO-A-89/01159
- WO-A-90/08783
- DE-A- 3 227 474
- DE-A- 4 024 528
- GB-A- 2 218 808

## Description

L'invention concerne un capteur moléculaire permettant de reconnaître spécifiquement des molécules, ces molécules pouvant être volatiles ou en solution ou en suspension en milieu aqueux.

Plus précisément, le capteur selon l'invention permet d'identifier toutes molécules (hydrocarbures, alcools, esters, cétones...) émanant de véhicules terrestres, marins ou aériens. Il utilise pour cela des anticorps spécifiques aux molécules à identifier et un procédé d'amplification enzymatique permettant de sensibiliser le capteur à de faibles teneurs en molécules à détecter.

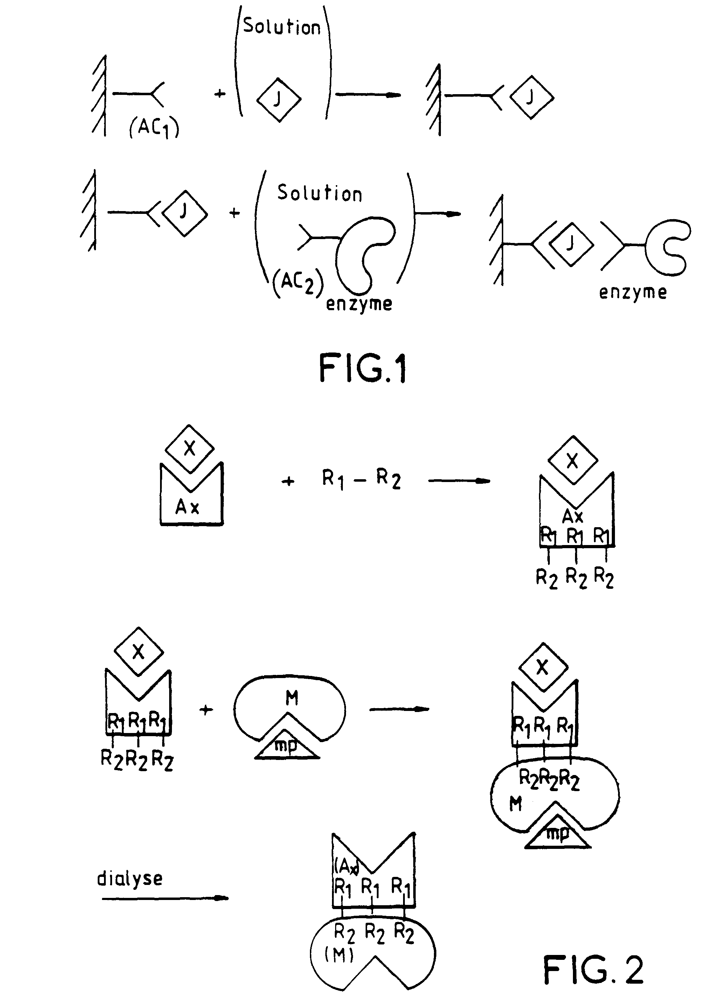

Il existe actuellement des tests de dépistage de molécules utilisant des procédés d'amplification enzymatique. Ces tests fonctionnent de la manière suivante :
- un anticorps spécifique (AC₁) de la molécule à détecter est fixé sur une membrane ; on plonge l'ensemble dans une solution contenant des molécules à détecter (J), permettant l'appariement de l'anticorps (AC₁) et de la molécule (J) l'appariement s'opérant à un endroit spécifique de ladite molécule. Dans un second temps l'ensemble AC₁-(J) peut être plongé dans une autre solution contenant un autre anticorps anti J spécifique AC₂ pouvant être lui-même fixé à une enzyme. On obtient alors la structure décrite à la figure 1. Ce groupement moléculaire peut via l'enzyme transformer un substrat S en produit P à une vitesse dépendant du pouvoir catalytique de l'enzyme (nombre de rotations). L'amplification enzymatique ainsi obtenue permet de produire une quantité détectable de produit P même lorsque les molécules à capter sont en très faibles concentrations. On peut typiquement faire apparaître une acidité, une basicité, une coloration ou tout autre phénomène physico-chimique. Un tel procédé de détection présente cependant les inconvénients d'opérer en solution et en deux temps : la capture de la molécule dans un premier bain puis dans un second bain, l'apparition de produit P générant la propriété physique ou chimique détectable.

La présente invention propose un capteur moléculaire utilisant un unique milieu réactionnel capable de piéger les molécules à détecter et de générer des propriétés physiques détectables. Plus précisément, l'invention propose un capteur moléculaire caractérisé en ce qu'il comprend un milieu réactionnel séparé d'un milieu extérieur par une interface (I) sur laquelle sont fixés des ensembles moléculaires qui résultent de l'association d'un anticorps (Ax) spécifique de la molécule à détecter (X) présente dans le milieu extérieur et d'une enzyme (E) apte à se déformer à l'issue de la fixation de la molécule (X). La déformation en question provoque l'activation de l'enzyme (E) dans le milieu réactionnel qui peut alors transformer un substrat S en un produit P. Cette transformation génère une modification de propriété du milieu, détectable par un transducteur (T) situé dans le milieu réactionnel.

L'enzyme possède de préférence un inhibiteur allostérique qu'elle relargue lors de la capture d'une molécule (X) par une molécule (Ax).

Pour réaliser le capteur moléculaire selon l'invention, l'interface peut avantageusement être une membrane et l'association de l'anticorps (Ax) et de l'enzyme (E) peut être obtenue par l'intermédiaire de réactifs bifonctionnels.

Le capteur selon l'invention peut avantageusement être un capteur moléculaire caractérisé en ce qu'il est l'assemblage de n capteurs (Ci), chaque capteur (Ci) ayant à son interface des ensembles (Gi) résultant de l'association d'un anticorps (Axi) et d'une enzyme (E), la lecture des n transducteurs (Ti) permettant d'identifier la composition d'un mélange de molécules différentes.

La présente invention sera mieux comprise et d'autres avantages apparaîtront à la lecture de la description qui va suivre et des figures annexées parmi lesquelles :
- la figure 1 illustre un schéma réactionnel utilisé dans un test de dépistage de molécule en solution, selon l'art connu ;
- la figure 2 illustre un exemple de schéma réactionnel permettant la réalisation de l'ensemble (G) ;
- la figure 3 illustre un schéma de capteur selon l'invention ;
- la figure 4 illustre un exemple de schéma réactionnel conduisant à l'apparition de produit (P) détecté par le transducteur (T).

Le capteur selon l'invention utilise une interface (I) comportant d'une part un anticorps (Ax) adapté à la molécule (X) que l'on veut détecter et d'autre part une enzyme (E) capable de générer des phénomènes physiquement détectables.

Dans un premier temps, il est donc nécessaire de fabriquer des anticorps spécifiques capables de s'accoupler avec des molécules (X) précises. De manière générale un anticorps est capable de reconnaître un antigène bien précis. Pour fabriquer cet anticorps on peut utiliser la méthode classique de production d'anticorps par voie animale. Cependant les molécules que l'on cherche à détecter sont souvent des molécules simples qu'il devient nécessaire d'associer à des molécules antigéniques pour parvenir à produire un anticorps de l'ensemble. Typiquement et à titre d'exemple on peut fixer la molécule sur l'albumine bovine (protéine porteuse), l'ensemble est inoculé à l'animal qui fabrique des anticorps contre l'ensemble protéine porteuse-antigène. Ceux-ci sont récupérés après deux à trois mois d'attente puis séparés. Il est ninsi possible de fabriquer un certain nombre d'anticorps spécifiques capables de reconnaître des parties différentes des molécules que l'on cherche à détecter. La figure 1 représentant une interaction molécule-anticorps schématise bien ce phénomène, dans cet exemple deux anticorps AC₁ et AC₂ interagissent avec des parties différentes de la molécule (J) à détecter.

L'anticorps (Ax) ainsi fabriqué est ensuite fixé par covalence à une enzyme (E) capable de se déformer lorsque la molécule (X) est captée. La déformation de l'enzyme (E) génère le phénomène physique que l'on veut mesurer. Pour cela on utilise par exemple une enzyme capable de devenir active lorsqu'elle est déformée. Dans le capteur selon l'invention l'ensemble (G) résultant de l'association de (Ax) et de (E) doit être fixé à l'interface milieu extérieur, milieu réactionnel. Cette interface peut être une membrane. L'ensemble (G) peut être obtenu par l'intermédiaire de réactifs bifonctionnels dont les deux fonctions sont adaptées d'une part à l'anticorps (Ax) et d'autre part à l'enzyme (E). Ces réactifs bifonctionnels jouent le rôle d'agrafes et assurent par liaison covalente l'association requise entre (Ax) et (E). Les anticorps de masse voisine de 100 000 sont ainsi associés à des enzymes (E) de masse également importante pouvant être de l'ordre de 50 000 et plus. Ainsi lorsqu'une petite molécule (X) rigide est captée par son anticorps (Ax) très volumineux et très plastique, elle génère la déformation de (Ax) qui elle-même entraîne la déformation de l'enzyme (E). L'accrochage entre (Ax) et (E) est toutefois très délicat dans la mesure où certains sites réactifs doivent être impérativement protégés pendant l'opération de fixation covalente. En effet l'enzyme (M) doit être accrochée à l'anticorps (Ax) en un site différent du site accepteur de la molécule à capter (X). Pour cela on suit le schéma réactionnel proposé à la figure 2. Dans un premier temps, on réalise l'accrochage d'une molécule (X) sur l'anticorps (Ax), cet accrochage étant de type non covalent ; puis on greffe des "agrafes" bifonctionnelles sur la partie libre de l'anticorps (Ax) les "agrafes" étant schématisées par des groupements R₁-R₂. On réalise alors l'accrochage de l'enzyme (E) sur le réactif R₂. L'ensemble peut ensuite être traité de manière à éliminer la molécule (X). Cette élimination peut être obtenue par dialyse, ultrafiltration ou tamisage moléculaire. L'enzyme (E) représente elle aussi une partie sensible capable d'être activée pour générer les propriétés physiques que l'on cherche à mesurer, révélatrices de la présence de molécules (X) dans le milieu extérieur. Pour cela on traite préalablement l'enzyme (E) de manière à lui accrocher en son site sensible une molécule protectrice (mₚ) par des liaisons de faible énergie, ainsi lors de l'accrochage de (Ax) et de (E) les sites sensibles de ces deux molécules se trouvent protégés. La molécule (mₚ) peut également être éliminée facilement par dialyse.

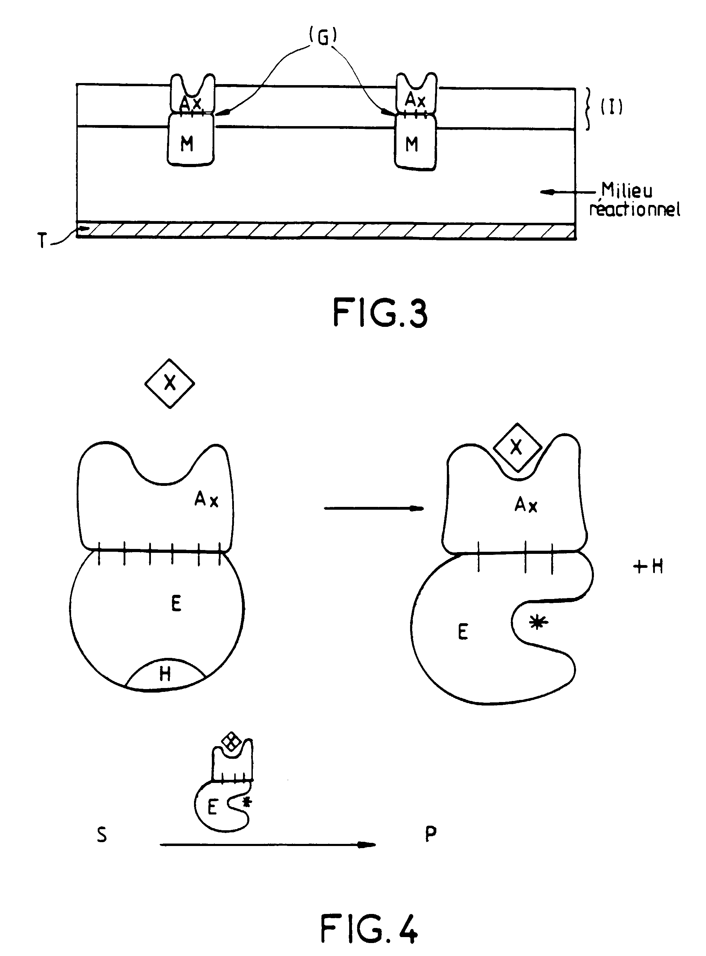

L'ensemble (G) résultant de la fixation de l'anticorps (Ax) et de l'enzyme (E) via des agrafes doit être inséré à l'interface séparant le milieu extérieur du milieu réactionnel Pour cela il est possible de réaliser une membrane très souple supportant l'ensemble (G) tout en étant capable de transmettre la déformation de l'anticorps (Ax) à l'enzyme (E) ; pour obtenir la membrane souhaitée, on petit avantageusement incuber l'ensemble (G) avec des phospholipides générant ainsi des films fins extrêmement souples. Cette membrane comprenant l'ensemble (G) peut alors être déposée à la surface du milieu réactionnel pour réaliser un capteur selon l'invention tel que celui représenté à la figure 3. Ce milieu réactionnel peut typiquement être une solution aqueuse contenant des sels, dont le pH est adapté à l'activité catalytique de l'enzyme dans le milieu.

Dans le milieu réactionnel l'enzyme (E) peut avantageusement être une enzyme possédant un inhibiteur allostérique de l'activité catalytique de ladite enzyme. Cet inhibiteur peut facilement être relargué dans le milieu réactionnel lors de la déformation de l'enzyme provoquée par la capture de la molécule (X). I,e site actif en question est donc démasqué par la déformation de l'enzyme lorsque la molécule (X) s'est accrochée à l'ensemble (G). La figure 4 illustre le schéma réactionnel correspondant à l'activation de l'enzyme provoquant la réaction désirée au niveau du substrat de la réaction (S) produisant le produit de la réaction (P) que l'on cherche à doser au niveau du transducteur (T) situé clans le milieu réactionnel.

L'exemple suivant de réalisation d'un capteur selon l'invention concerne la détection d'hydrocarbures et d'autres substances pouvant émaner d'engins divers.

Ces hydrocarbures ou autres substances peuvent être de type polyaromatique. Il convient dans un premier temps de fabriquer la molécule (Ax). Pour cela on peut greffer sur une substance polyaromatique une fonction NH₂ qui permettra son accrochage sur la serum albumine bovine. L'ensemble est administré à un lapin sous forme d'injections (2 à 3 injections à quelques semaines d'intervalle). Au bout de quelques mois on prélève du sang de lapin, dont on élimine les globules rouges pour recueillir le serum. Les nombreux anticorps ainsi développés au sein de l'animal peuvent être séparés par exemple par la chromatographie d'affinité. Pour cela on greffe la molécule (X) soit la substance polyaromatique sur un support fixe (type résine carboxylique) ; mis en contact avec tous les anticorps, ces derniers viennent sélectivement s'apparier à la molécule (X) en des endroits privilégiés de cette molécule. On parvient ainsi à des billes insolubles dans le milieu, auxquelles sont accrochés les anticorps. En opérant une séparation par un gradient de sel (type NaCl) on obtient les différents anticorps purifiés.

L'anticorps (Ax) ainsi élaboré peut être associé à une molécule (M) déterminée. On peut avantageusement utiliser une enzyme allostérique comme molécule (M). L'association de (Ax) et de (M) peut se faire par l'intermédiaire de réactifs bifonctionnels tels que par exemple le dimethyl suberimidate-2HCl (DMS) de formule chimique :

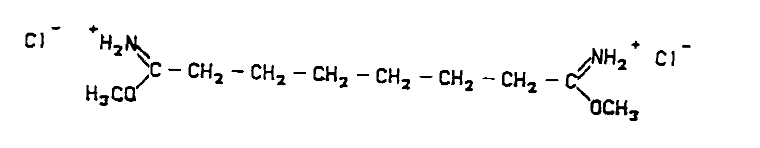

L'ensemble (G) ainsi élaboré est mis en présence de phospholipides pour réaliser une membrane présentant d'un côté des anticorps (Ax) de l'autre des molécules d'enzymes.

L'enzyme choisie doit être capable de relarguer un inhibiteur lors de sa déformation. Activée cette enzyme doit posséder une activité catalytique et transformer du substrat (S) un ou des produits (P) présentant une propriété physique facilement détectable et mesurable. L'enzyme homosérine acetyl transférase répond particulièrement bien à tous ces critères. Elle possède un inhibiteur (H) : la methionine qu'elle peut relarguer pour faire apparaître un site capable de transformer un certain nombre de substrats de réaction en produits de réaction. Ces substrats sont S₁ : l'homosérine et S₂ la succinyl coenzyme A dont les produits de réaction sont respectivement P₁ : l'O-succinyl-homosérine et P₂ le coenzyme A. L'avantage du produit P₂ est qu'il est capable de faire apparaître une coloration violette au niveau d'une membrane sur laquelle est fixé de l'acide dithionitrobenzoïque. En effet la dégradation de l'acide par le produit P₂ génère la coloration dont l'intensité peut aisément être enregistrée par une petite cellule de colorimétrie. Le milieu réactionnel comportant tous ces produits peut être un tampon phosphate dont le pH est typiquement compris entre 4 et 7,5.

Ainsi dans ce capteur moléculaire de substances polyaromatiques, le nombre de molécules captées au niveau des anticorps (Ax) est relié à l'intensité de coloration mesurée par la cellule de colorimétrie.

Pour identifier la composition d'un mélange de molécules de nature différente, il est possible de réaliser un assemblage de capteurs selon l'invention, la lecture de chaque transducteur (Ti) relatif à une molécule particulière (Xi) permettant d'évaluer les quantités respectives de molécules piégées et ainsi d'en déduire la composition du mélange à identifier. On peut avantageusement utiliser le même milieu réactionnel et les mêmes transducteurs (Ti), chaque capteur devant néanmoins être séparé de son voisin pour ne pas opérer une mesure globale de l'ensemble des molécules du mélange.

Dans le cas présent, le balayage par une cellule de colorimétrie des différentes membranes colorées permet d'identifier la composition du mélange.

## Revendications

1. Capteur moléculaire caractérisé en ce qu'il comprend un milieu réactionnel séparé d'un milieu extérieur par une interface (I) sur laquelle sont fixés des ensembles moléculaires qui résultent de l'association d'un anticorps (Ax) spécifique de la molécule à détecter (X) présente dans le milieu extérieur et d'une enzyme (E) apte à se déformer à l'issue de la fixation de la molécule (X), la déformation en question provoquant l'activation de l'enzyme (E) dans le milieu réactionnel avec comme produit de réaction, une substance générant une propriété physique détectable par un transducteur (T) situé dans le milieu réactionnel.

2. Capteur selon la revendication 1, caractérisé en ce que l'enzyme possède un inhibiteur allostérique qu'elle relargue lors de la capture d'une molécule (X) par une molécule (Ax).

3. Capteur selon l'une des revendications 1 ou 2, caractérisé en ce que l'interface (I) est une membrane.

4. Capteur selon l'une des revendications 1 à 3, caractérisé en ce que l'association d'une molécule (Ax) et d'une enzyme (E) est assurée par un réactif bifonctionnel.

5. Capteur selon l'une des revendications 1 à 4, caractérisé en ce que l'enzyme est l'homosérine acetyl transferase.

6. Capteur selon la revendication 5, caractérisé en ce que le transducteur (T) est une cellule de colorimétrie.

7. Capteur moléculaire caractérisé en ce qu'il est l'assemblage de n capteurs (Ci) selon la revendication 1, chaque capteur (Ci) ayant à son interface un ensemble (Gi) résultant de l'association d'un anticorps (Axi) et d'une enzyme (E), la lecture des n transducteurs (Ti) permettant d'identifier la composition d'un mélange de molécules différentes.

## Patentansprüche

1. Molekülsonde, dadurch gekennzeichnet, daß sie ein Reaktionsmilieu enthält, das von einem Außenmilieu durch eine Übergangsschicht (I) getrennt ist, auf der Molekülgruppen fixiert sind, die aus der Paarung eines spezifischen Antikörpers (Ax) des in dem äußeren Milieu vorhandenen und nachzuweisenden Moleküls (X) und eines Enzyms (E) resultiert, das sich nach der Fixierung des Moleküls (X) umformen kann, wobei diese Umformung eine Aktivierung des Enzyms (E) in dem Reaktionsmilieu mit einer Substanz als Reaktionsprodukt hervorruft, die eine durch einen in diesem Reaktionsmilieu liegenden Transduktor (T) nachweisbare physikalische Eigenschaft erzeugt.

2. Sonde nach Anspruch 1, dadurch gekennzeichnet, daß das Enzym einen allosterischen Inhibitor enthält, der freigegeben wird beim Einfangen eines Moleküls (X) durch ein Molekül (Ax).

3. Sonde nach einem der Ansprüche 1 und 2, dadurch gekennzeichnet, daß die Übergangsschicht (I) eine Membran ist.

4. Sonde nach einem der Ansprüche 1 bis 3, dadurch gekennzeichnet, daß die Paarung eines Moleküls (Ax) und eines Enzyms (E) durch einen bifunktionalen Reaktionsstoff bewirkt wird.

5. Sonde nach einem der Ansprüche 1 bis 4, dadurch gekennzeichnet, daß das Enzym von Homoserinacetyltransferase gebildet wird.

6. Sonde nach Anspruch 5, dadurch gekennzeichnet, daß der Transduktor (T) eine Farbmeßzelle ist.

7. Molekülsonde, dadurch gekennzeichnet, daß sie von einer Kombination von n Sonden (Ci) nach Anspruch 1 gebildet wird, wobei jede Sonde (Ci) an ihrer Übergangsschicht eine Gruppierung (Gi) hat, die aus der Paarung eines Antikörpers (Axi) und eines Enzyms (E) resultiert, wobei das Auslesen von n Transduktoren (Ti) die Identifizierung der Zusammensetzung einer Mischung unterschiedlicher Moleküle erlaubt.

## Claims

1. Molecular sensor, characterized in that it comprises a reaction medium separated from an external medium by an interface (I) on which are fixed molecular assemblies which result from the combination of a specific antibody (Ax) of the molecule to be detected (X) present in the external medium and of an enzyme (E) capable of changing its shape as a result of the fixing of the molecule (X), the change of shape in question causing the activation of the enzyme (E) in the reaction medium with, as reaction product, a substance which generates a physical property detectable by a transducer (T) situated in the reaction medium.

2. Sensor according to Claim 1, characterized in that the enzyme has an allosteric inhibitor which it releases during the capture of a molecule (X) by a molecule (Ax).

3. Sensor according to one of Claims 1 and 2, characterized in that the interface (I) is a membrane.

4. Sensor according to one of Claims 1 to 3, characterized in that the combination of a molecule (Ax) and of an enzyme (E) is provided by a bifunctional reactant.

5. Sensor according to one of Claims 1 to 4, characterized in that the enzyme is homoserine acetyl transferase.

6. Sensor according to Claim 5, characterized in that the transducer (T) is a colorimetric cell.

7. Molecular sensor, characterized in that it is the assembly of n sensors (Ci) according to Claim 1, each sensor (Ci) having, at its interface, an assembly (Gi) resulting from the combination of an antibody (Axi) and of an enzyme (E), it being possible to identify the composition of a mixture of different molecules by reading n transducers (Ti).
